# EUROPEAN PATENT SPECIFICATION

(11) **EP 4 358 905 B1**
(45) Date of publication and mention of the grant of the patent: **31.12.2025**
(21) Application number: 22741940.5
(22) Date of filing: 22.06.2022
(51) Int. Cl.: A61F 5/44, A61F 5/451, A61F 5/455, A61F 5/442

(54) **URINE COLLECTION SYSTEMS HAVING A CLEANING SYSTEM, AND RELATED METHODS**
URINSAMMELSYSTEME MIT EINEM REINIGUNGSSYSTEM UND ZUGEHÖRIGE VERFAHREN
SYSTÈMES DE COLLECTE D'URINE AYANT UN SYSTÈME DE NETTOYAGE, ET PROCÉDÉS ASSOCIÉS

(30) Priority: 24.06.2021 US 202163214551 P
(43) Date of publication of application: 01.05.2024
(73) Proprietor: Purewick Corporation, El Cajon, California 92020 (US)
(72) Inventor: BARNES, Nathaniel, Covington, Georgia 30016 (US); LEWIS, Briana, Forest Park, Georgia 30297 (US)
(74) Representative: Studio Torta S.p.A.
(86) International application number: PCT/US2022/034457
(87) International publication number: WO 2022/271783

(56) References cited:
- EP-A1- 3 788 992
- WO-A1-2022/150463
- WO-A2-2022/076427
- US-A- 4 957 487
- US-A1- 2012 066 825
- US-A1- 2012 233 761

## Description

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections. Conventional urine collection devices also may be limited to use when a patient is confined to a bed in a supine position.

An example is provided by US 4957487, which discloses an external male urinary catheter and collection system that utilizes a sheath-like member for accepting the male penis, a negative pressure evacuation device communicating with the sheath-like member, the negative pressure causing the penis to elongate in a simulated erection, and a thin-walled sealing portion of the sheath-like member to provide an air-tight seal about the elongated penis. A one-way drainage tube to permit draining of urine into the evacuation device and a one-way air tube to permit equalization of the negative pressure between the sheath-like member and the evacuation device after urine has been voided into the evacuation device are also provided. The evacuation device may be worn attached to the leg of the wearer.

In this connection, EP 3788992 A1 relates to an automatic excretion disposal device which is configured to automatically perform an excretion disposal of a person to be cared, the device including: a cup which is attached to a body of the person to be cared to receive excretion; a detection unit which is configured to detect excretion in the cup; a feed water pipe which is configured to decompress cleaning water from a water supply to a predetermined pressure and directly supplies the cleaning water, on the basis of detection result of the detection unit; a suction motor which is configured to suck sewage generated when the body and inside of the cup are cleaned with the cleaning water supplied from the feed water pipe, and to discharge the sewage to outside of the cup; a tank which is configured to temporarily store the drainage sucked by the suction motor; and a drainage pump which is configured to cause the drainage to forcibly flow out to a drainage piping connected to a sewer or a septic tank, while pulverizing solid matters contained in drainage in which the sewage stored in the tank is mixed with the cleaning water directly supplied from the feed water pipe.

In this respect, WO 2022/150463 A1 concerns a portable urine collection system that includes a urine collection device, a conduit in fluid communication with the urine collection device, a urine collection container having an interior region, a pump, and a container support. The pump is in fluid communication with the urine collection container and is configured to pull a vacuum on the interior region of the urine collection container effective to draw urine from the urine collection device through the conduit into the urine collection container. The container support is configured to detachably secure to a wheelchair and support at least the urine collection container.

For example, WO 2022/076427 A2 relates to a fluid transfer assembly for the collection and disposal of urine including a urine collection device and a cleaning device. The urine collection device including a container configured to store urine collected from the urine collection device and an outlet configured to expel the urine from the container. The cleaning device can be in fluid communication with the container, and include a chamber configured to store a liquid and a pump configured to modulate pressure within the container of the urine collection device.

Thus, users and manufacturers of fluid collection devices continue to seek new and improved devices, systems, and methods to collect urine.

### SUMMARY

The present invention discloses a portable urine collection system and a method of cleaning a canister of a portable urine collection system according to the appended claims.

### BRIEF DESCRIPTION OF THE DRAWINGS

The drawings illustrate several embodiments of the present disclosure, wherein identical reference numerals refer to identical or similar elements or features in different views or embodiments shown in the drawings.
- **FIG. 1A** is a block diagram of a portable urine collection system, according to an embodiment.
- **FIG. 1B** is a side view of a urine collection system in a urine collection configuration, according to an embodiment.
- **FIG. 1C** is a side view of the urine collection system of **FIG. 1B** in a urine removal configuration, according to an embodiment.
- **FIG. 1D** is a side view of the urine collection system of **FIG. 1B** in a cleaning configuration, according to an embodiment.
- **FIG. 2A** is a side view of a urine collection system in a urine collection and removal configuration, according to an embodiment.
- **FIG. 2B** is a side view of the urine collection system of **FIG. 2A** in a cleaning configuration, according to an embodiment.
- **FIG. 3** is a flow diagram of a method of cleaning a urine collection system, according to an embodiment.

### DETAILED DESCRIPTION

Embodiments disclosed herein are related to urine collection systems having a cleaning apparatus or system, and methods of using the same. The devices and systems disclosed herein are configured to collect fluids from an individual. The fluids collected by the fluid collection devices may include at least one of urine, vaginal discharge, penile discharge, reproductive fluids, blood, sweat, or other bodily fluids. In many embodiments, the fluids collected by the fluid collection device are drawn into a urine collection container. Conventional urine collection systems typically require a user lift and remove the urine collection container from the urine collection system to empty and clean the urine collection system. Moreover, conventional urine collection systems also may require a user to remove a lid from the urine collection container to empty and clean the urine collection container. In addition to the inconvenience of these activities, these activities also increase the likelihood of the spilling urine collected in the urine collection container in undesired locations, increasing the likelihood of unsanitary conditions.

Urine collection systems described herein may be adjustable between a urine collection configuration, a urine removal configuration, and a cleaning configuration. For example, in the cleaning configuration, at least one, some, or all embodiments of urine collection systems described herein provide the technical effect of facilitating cleaning of the urine collection container without removing the urine collection container from the urine collection system and without removing the lid from the urine collection container. Moreover, in the removal configuration, at least one, some, or all embodiments of urine collection systems described herein provide the technical effect of allowing removal of urine or other fluids from the urine collection container without removing the urine collection container from the urine collection system and without removing the lid from the urine collection container. Removal of urine from the urine collection container and also cleaning of the urine collection container without removal of the urine collection container from the urine collection system and/or removal of the lid from the urine collection container promotes sanitary conditions around the user.

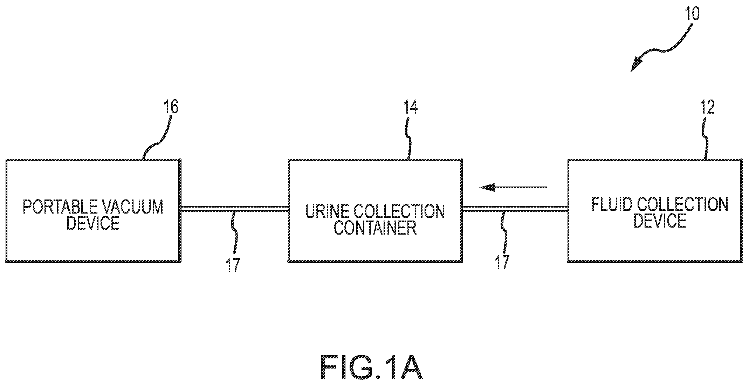

**FIG. 1A** is a block diagram of a fluid collection system 10, according to an embodiment. The fluid collection system 10 may be included in embodiments of fluid collection systems described herein. The system 10 includes a fluid collection device 12 (*e.g.,* any of the fluid collection devices disclosed herein), a urine collection container 14, and a pump 16 (*e.g.,* portable vacuum device or source). The fluid collection device 12, the urine collection container 14, and the pump 16 may be fluidly coupled to each other via one or more conduits 17. For example, fluid collection device 12 may be operably coupled to one or more of the urine collection container 14 or the pump 16 via the conduit 17. In some embodiments, the pump 16 may be secured directly to the urine collection container 14. Fluid (*e.g.,* urine or other bodily fluids) collected in the fluid collection device 12 may be removed from the fluid collection device 12 via the conduit 17 secured to the fluid collection device 12. Suction force may be introduced into the chamber of the fluid collection device 12 via the inlet of the conduit 17 responsive to suction (*e.g.,* vacuum) force applied at the outlet of the conduit 17.

The suction force may be applied to the outlet of the conduit 17 by the pump 16 either directly or indirectly. The suction force may be applied indirectly via the urine collection container 14. For example, the outlet of the conduit 17 may be disposed within or fluidly coupled to an interior region of the urine collection container 14 and an additional conduit 17 may extend from the urine collection container 14 to the pump 16. Accordingly, the pump 16 may apply suction to the fluid collection device 12 via the urine collection container 14. The suction force may be applied directly via the pump 16. For example, the outlet of the conduit 17 may be disposed within the pump 16. An additional conduit 17 may extend from the pump 16 to a point outside of the fluid collection device 12, such as to the urine collection container 14. In such examples, the pump 16 may be disposed between the fluid collection device 12 and the urine collection container 14.

The urine collection container 14 is sized and shaped to retain a fluid therein. The urine collection container 14 may include a bag (*e.g.,* drainage bag), a bottle, a canister, or a cup (*e.g.,* collection jar), or any other enclosed container for storing bodily fluid(s) such as urine. In some examples, the conduit 17 may extend from the fluid collection device 12 and attach to the urine collection container 14 at a first point therein. An additional conduit 17 may attach to the urine collection container 14 at a second point thereon and may extend and attach to the pump 16. Accordingly, a vacuum (*e.g.,* suction) may be drawn through fluid collection device 12 via the urine collection container 14. Fluid, such as urine, may be drained from the fluid collection device 12 using the pump 16.

The pump 16 may include one or more of a manual vacuum pump, and electric vacuum pump, a diaphragm pump, a centrifugal pump, a displacement pump, a magnetically driven pump, a peristaltic pump, or any pump configured to produce a vacuum or suction force. The pump 16 may provide a vacuum or suction to remove fluid from the fluid collection device 12. In some examples, the pump 16 may be powered by one or more of a power cord (*e.g.,* connected to a power socket), one or more batteries, or even manual power (*e.g.,* a hand operated vacuum pump). In some examples, the pump 16 may be sized and shaped to fit outside of, on, or within the fluid collection device 12. For example, the pump 16 may include one or more miniaturized pumps or one or more micro pumps. The vacuum sources disclosed herein may include one or more of a switch, a button, a plug, a remote, or any other device suitable to activate the pump 16.

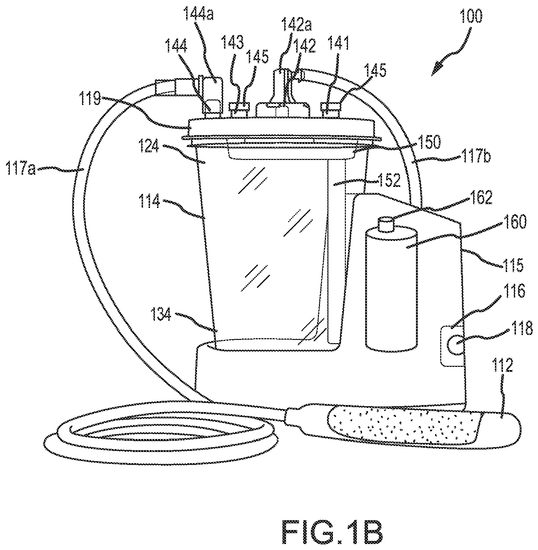

**FIG. 1B** shows an example of a urine collection system 100 in a urine collection configuration, according to an embodiment. The urine collection system 100 may include a urine collection device 112, a first conduit 117a in fluid communication with an interior region of the urine collection device 112, a urine collection container, and a base 115 having a pump 116 positioned thereon or secured therein (*e.g*., at least partially positioned within a housing of the base 115). The urine collection container may include a canister 114 and a lid 119 selectively secured or securable to the canister 114. The canister 114 may include a top region 124 and a bottom region 134 and may at least partially define an interior region in fluid communication with the internal region of the urine collection device 112 via the first conduit 117a. The canister 114 may be reusable and dishwasher safe, and may include a generally rigid material such as polycarbonate or glass. In some embodiments, the canister 114 may be disposable. The urine collection system 100 may be configured to accommodate different volumetric sizes of canisters 114. The canister 114 may be substantially cylindrical or frustoconical. In some embodiments, the canister may include other shapes and configurations, such as a generally square or rectangular side profile.

The urine collection device 112 may be configured to be positioned at least proximate to a urethra of a user. While the urine collection device 112 shown in **FIG. 1B** includes a female urine collection device, the urine collection device 112 may instead include a male urine collection device. PCT International Application No. PCT/US2019/029616, for example, describes various embodiments of both male and female fluid collection devices. Moreover, the urine collection device 112 may be interchangeable in the urine collection system 100 between different types, varieties, and sizes of male or female urine collection devices. Generally, the urine collection device 112 may include a surface sized to be positioned proximate or adjacent to the urethra and configured to wick urine or other fluids away from the user. Urine or other fluids may be wicked from the surface to a reservoir in the urine collection device 112.

The urine collection system 100 also includes the first conduit 117a in fluid communication with an interior region (*e.g*. reservoir) of the urine collection device 112 and the interior region of the canister 114. The first conduit 117a may be positioned between the urine collection device 112 and the canister 114. The urine collection system 100 also may include the second conduit 117b providing fluid communication between the pump 116 and the interior region of the canister 114. In some embodiments, the pump 116 may be secured directly to the canister 114, and the second conduit 117b may be absent from the urine collection system 100. The conduits 117a, 117b may include a flexible tube. In some embodiments, at least a portion of the first conduit 117a is substantially opaque, thereby inhibiting viewing of the urine within the first conduit 117a.

The pump 116 is in fluid communication with the interior region of the canister 114 and is configured to pull a vacuum on the interior region of the canister 114 effective to draw the urine from the urine collection device 112 through the first conduit 117a into the canister 114. The pump 116 may be secured directly to the canister 114, or the conduit 117b may fluidly couple the pump 116 with the interior region of the canister 114. The pump 116 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 116 may provide a vacuum or suction to remove fluid from the fluid collection device 112. In some examples, the pump 116 may be powered by one or more batteries or other power sources. The pump 116 may be included in a housing that also supports the canister 114. In some examples, the pump 116 may be sized and shaped to fit within a container support on a wheelchair. In some embodiments, the pump 116 may include a wall-mounted pump. The pump 116 may include one or more of a switch, a button, a plug, a remote, or any other device suitable to activate the pump 116.

In some embodiments, urine collection systems described herein are configured to clean inside the canister 114 and the lid 119. To facilitate this cleaning inside the canister 114 and the lid 119, the lid 119 may include multiple ports and a cleaning solution dispenser 150 positioned or positionable in the interior region of the canister 114, according to some embodiments. For example, the lid 119 may include multiple ports 141, 142, 143, 144 for attachment of the conduits 117a, 117b thereto. One or more of the multiple ports 141, 142, 143, 144 may include an adapter (*e.g.*, adapters 142a, 144a) detachably secured or securable thereto that improves attachments of the conduits 117a, 117b to the multiple ports 141, 142, 143, 144. In some embodiments, the lid 119 may include additional or fewer ports or adapters than those shown in **FIG. 1B****.** Except for the ports and/or adapters for attachment of the conduits 117a, 117b, the canister 114 may be sealed and airtight such that the urine collected in the canister 114 does not leak or spill.

In some embodiments, the lid 119 may include a urine port 144 to which an outlet of the first conduit 117a may be detachably secured (either directly or via an adapter 144a secured to the urine port 144) to provide fluid communication between urine collection device 112 and the interior region of the canister 114. A second conduit 117b may provide fluid communication between the interior region of the canister 114 and the pump 116. For example, the lid 119 may include a vacuum port 142 to which an outlet of the second conduit 117b may be detachably secured (either directly or via an adapter 142a secured to the vacuum port 142) to provide fluid communication between the pump 116 and the interior region of the canister. The vacuum port 142 and/or the urine port 144 may include adapters 142a, 144a, respectively, detachably secured thereto to secure the respective conduit 117b, 117a to the vacuum port 142 and/or the urine port 144. In some embodiments, at least one (*e.g*., both) of the first conduit 117a and the second conduit 117b may be detachably connected or secured to the canister 114 effective to provide fluid communication with the interior region of the canister 114. For example, the canister may include at least one (*e.g.* both) of the urine port 144 and the vacuum port 142. When the lid 119 is secured to the canister 114, an inlet of the first conduit 117a may be secured to the urine collection device 112, the outlet of first conduit may be secured to the urine port 144, the second conduit 117b may be secured to the vacuum port 142, and activation of the pump may create a negative pressure in the interior region of the canister 114 effective to draw urine in the urine collection device 112 through the first conduit 117a into the interior region of the canister 114.

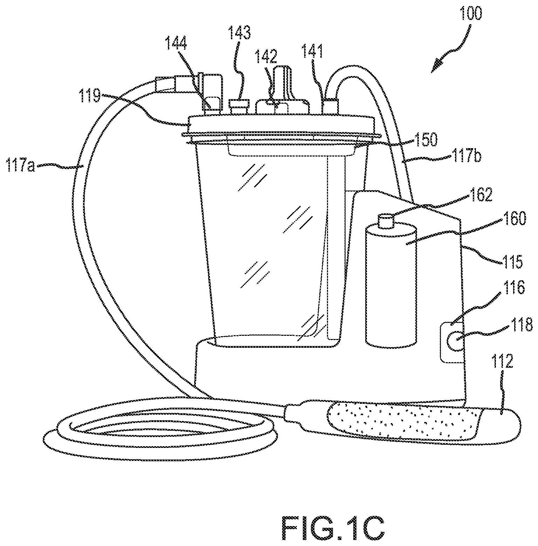

In some embodiments, the urine collection system 100 also may be configured to draw and remove at least some (*e.g*. all) of the urine held in the interior region of the canister 114. For example, one of the canister 114 or the lid 119 may include a removal port 141 positioned to have urine withdrawn from the canister 114 therethrough. In **FIG. 1B****,** the removal port 141 is shown capped or closed on the lid 119 with a cap 145 that is removably secured to the removal port 143. When the cap 145 is secured to the removal port 141, the cap 145 prevents fluid communication with the interior region of the canister 114 through the dispenser port 141. Turning to **FIG. 1C****,** when the urine collection system 100 is adjusted to the urine removal configuration, the removal port 141 may be positioned on the lid 119 and/or sized to have the second conduit 117b (or a different conduit) removably secured thereto when the cap 145 is removed from the removal port 141. In some embodiments, the removal port 141 may be positioned on the canister 114, such as on the bottom region 134 of the canister 114.

The urine collection system 100 also may include an interior conduit 152 positioned or positionable in the interior region of the canister 114. For example, the interior conduit 152 may be secured or securable to the canister 114 and/or the lid 119 effective to provide fluid communication with the removal port 141. The interior conduit 152 may include a tube or straw and may extend from the removal port 141 or the lid 119 at least partially into the interior region of the canister 114. In some embodiments, the interior conduit 152 extends from the removal port 141 into interior region of the bottom region 134 of the canister 114. For example, the interior conduit 152 may terminate at an inlet that is proximate the bottom of the canister 114. In some embodiments, the inlet of the interior conduit 152 may be positioned less than about 1 cm, less than about 5 mm, less than about 3 mm, or less than about 2 mm from the bottom of the canister 114. In some embodiments, the interior conduit 152 is flexible and configured to bend and lay on the bottom of the canister 114. The interior conduit 152 may be detachably secured or securable to an inner region of the lid 119 effective to provide fluid communication between the removal port 141 and the interior conduit 152.

In some embodiments, the pump 116 and/or the base 115 may include a discharge port 118 (*e.g*., exhaust port or opening) positioned and configured to discharge fluid (*e.g.* air or urine) drawn from the canister 114. For example, when the lid 119 is secured to the canister 114 and the second conduit 117b is secured to the vacuum port 142 (shown in **FIG. 1B****)**, activation of the pump 116 may draw air from within the canister 114 through the vacuum port 142, the second conduit 117b, and the pump 116, then discharge or exhaust the air through the discharge port 118. When the lid 119 is secured to the canister 114 and the second conduit 117b is secured to the removal port 141, activation of the pump 116 draws fluid (*e.g*., urine or air) in the canister 114 through the interior conduit 152, the removal port 141, and the second conduit 117b, and then discharge the fluid through the discharge port 118 on the base 115. An additional conduit (not shown) may be secured to the discharge port 118 to direct the discharged fluid to a desired location, such as a toilet.

In at least one, some, or all embodiments, the urine collection system 100 is configured to provide the technical effect of cleaning the interior of the canister 114 and/or the lid 119. For example, the canister 114 and/or the lid 119 may be cleaned after the fluid is removed from the canister 114. In some embodiments, the urine collection system 100 may include a cleaning apparatus or assembly with the canister 114 and/or the lid 119 that is adapted to clean the interior of the canister 114 and/or the lid 119. In some embodiments, the cleaning system may include a cleaning solution container 160 having a cleaning port 162, a dispenser port 143 on one of the lid 119 or the canister 114, and a dispenser 150 positioned or positionable in the interior region of the canister 114. The cleaning solution container 160 may hold a cleaning solution therein and may be detachably or fixedly secured to one of the base 115 or the canister 114. In some embodiments, the cleaning solution container 160 is separate (*e.g.*, not secured to the canister 114 or the base 115).

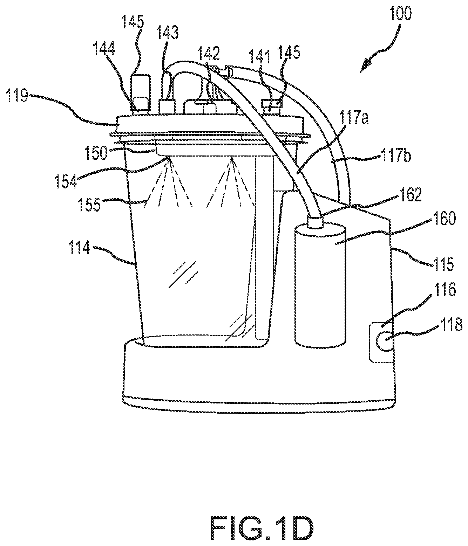

In **FIG. 1C****,** the dispenser port 143 is shown capped or closed with a cap 145 that is removably secured to the dispenser port 143. When the cap 145 is secured to the dispenser port 143, the cap 145 prevents fluid communication with the interior region of the canister 114 through the dispenser port 143. Turning to **FIG. 1D**, when the urine collection system 100 is adjusted to the cleaning configuration, the dispenser port 143 may be positioned on the lid 119 and/or sized to have the first conduit 117a (or a different conduit) removably secured thereto when the cap 145 is removed from the dispenser port 143. In some embodiments, the dispenser port 143 may be positioned on the canister 114, such as on a sidewall of the canister 114.

The urine collection system 100 also may include the dispenser 150 positioned or positionable in the interior region of the canister 114. For example, the dispenser 150 may be secured or securable to the canister 114 and/or the lid 119 effective to provide fluid communication with the dispenser port 143. In some embodiments, the dispenser 150 is detachably or fixedly secured to the lid 119 in fluid communication with the dispenser port 143. The dispenser 150 may include one or more openings 154 configured to dispense the cleaning solution 155 therethrough. For example, the dispenser 150 may include one or more misters or one or more spray nozzles configured to mist or spray the cleaning solution 155 into the interior region of the canister 114.

The outlet of the first conduit 117a may be detached from the urine port 144 and secured to the dispenser port 143 effective to provide fluid communication with the dispenser 150 through the dispenser port 143. The inlet of the first conduit 117a may be detached from the urine collection device 112 and secured to the cleaning port 162 effective to provide fluid communication between the interior region of the cleaning solution container 160 and the dispenser 150. A cap 145 may be secured to the urine port 145 after the first conduit 117a is removed effective to prevent fluid communication with the interior region of the canister 114 through the urine port 144. In some embodiments, an additional conduit different than the first conduit 117a may include an outlet secured or securable to the dispenser port 143 and an inlet secured or securable the cleaning port 162 effective to provide fluid communication between the cleaning solution container 160 and the dispenser 150. In operation, when the lid 119 is secured to the canister 114, the first conduit 117a is secured to the dispenser port 143 and the cleaning port 162, and the second conduit 117b is secured to the vacuum port 142 (or the removal port 141), activation of the pump 116 creates the negative pressure in the interior region of the canister 114 effective to draw cleaning solution 155 in the cleaning solution container 160 through the first conduit 117a, the dispenser port 143, and the one or more openings 154 of the dispenser 150 to dispense the cleaning solution 155 into the interior region of the canister 114.

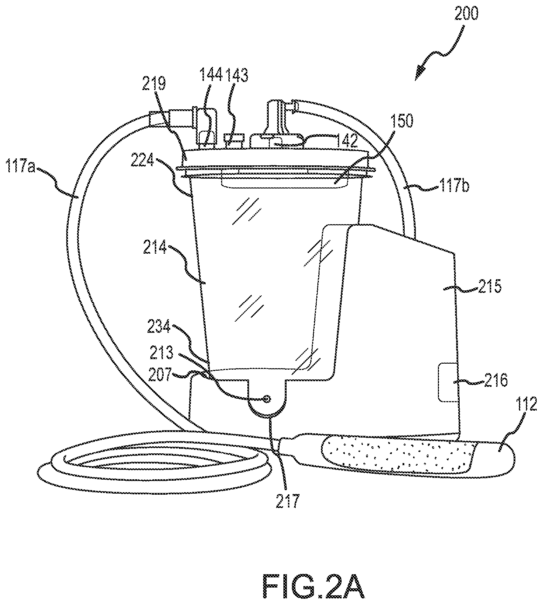

Turning now to **FIG. 2A****,** in some embodiments, urine may be removed from a urine collection system 200 without a pump 116, according to an embodiment. Unless otherwise noted, the urine collection system 200 may include any aspect of the urine collection system 100. For example the urine collection system 200 may include a base 215 having a pump 216 positioned therein and/or secured thereto. The urine collection system 200 also may include a urine collection container having a canister 214 and a lid 219. The canister 214 may include a top region 224 and a bottom region 234, and the lid 219 may include the vacuum port 142, the dispenser port 143, and the urine port 144. The removal port 141 may be absent from the lid 219. The urine collection system 200 may include the dispenser 150 secured or securable to at least one of the lid 219 or the canister 214. Unless otherwise noted, the canister 214, the base 215, the pump 216, the lid 219, and the dispenser 150 may include any aspect of the canister 114, the base 115, the pump 116, the lid 119, and the dispenser 150 described above in relation to the urine collection system 100.

**FIG. 2A** shows the urine collection system 200 in a urine collection configuration. In some embodiments, the canister 214 includes an opening 213 at the bottom region 234 of the canister 214. A plug or cap may be detachably secured or securable to the opening 213 to prevent fluid communication through the opening 213. When the plug is removed from the opening 213 to adjust the urine collection system to a urine removal configuration, fluid in the canister 214 may drain from the canister 214 through the opening 213. In some embodiments, one or more of the ports 142, 143, 144 may be also be opened when the cap is removed from the opening 213 to allow air to enter the interior region of the canister 214 for more effecting draining of the canister 214.

In some embodiments, the base 215 includes a recess 207 sized to receive at least a portion of the bottom region 234 of the canister 214. The base 215 also may include a notch 217 positioned to provide access to the cap and/or the opening 213 in the canister 214 when the canister 214 is positioned in the recess 207 of the base 215. In some embodiments, the canister 214 may include a port (*e.g*., the opening 213 may be configured as a port) sized and dimensioned for a conduit to detachably secure thereto. In some embodiments, the opening 213 includes a valve configured to prevent fluid communication through the opening 213 when no conduit is secured thereto and configured to allow fluid communication through the opening 213 when a conduit is secured thereto. The conduit secured to the opening 213 may allow a user to drain the canister 214 into a desired location, such as a toilet.

The urine collection system 200 also may be configured to clean the interior of the canister 214 and/or the lid 219. For example, the canister 214 and/or the lid 219 may be cleaned after the fluid is removed from the canister 214. In some embodiments, the urine collection system 200 may include a cleaning apparatus or assembly with the canister 214 and/or the lid 219 that is adapted to clean the interior of the canister 214 and/or the lid 219. In some embodiments, the cleaning system may include a cleaning solution container 260 having a cleaning port 262, the dispenser port 143 on one of the lid 219 or the canister 214, and the dispenser 150 positioned or positionable in the interior region of the canister 214. The cleaning solution container 260 may hold a cleaning solution therein and may be separate from (*e.g*., not secured to) the canister 214 or the base 215. In some embodiments, the cleaning solution container 260 may be detachably or fixedly secured to one of the base 215 or the canister 214.

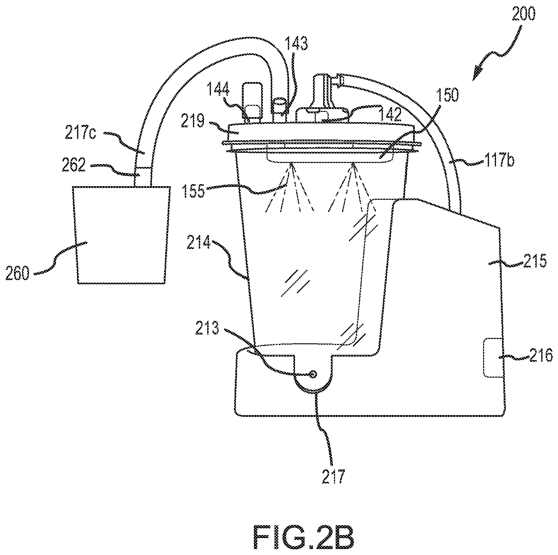

In **FIG. 2A****,** the dispenser port 143 is shown capped or closed with a cap 145 that is removably secured to the dispenser port 143. When the cap 145 is secured to the dispenser port 143, the cap 145 prevents fluid communication with the interior region of the canister 214 through the dispenser port 143. Turning to **FIG. 2B****,** the urine collection system 200 may be adjusted to a cleaning configuration having an additional conduit 217c (or the first conduit 217a) removably secured to the dispenser port 143 when the cap 145 is removed from the dispenser port 143. In some embodiments, the dispenser port 143 may be positioned on the canister 214, such as on a sidewall of the canister 214.

The outlet of the first conduit 117a may be detached from the urine port 144 and an outlet of the additional conduit 217c secured to the dispenser port 143 effective to provide fluid communication with the dispenser 150 through the dispenser port 143. The inlet of the additional conduit 217c may be secured to the cleaning port 262 effective to provide fluid communication between the interior region of the cleaning solution container 260 and the dispenser 150. A cap 145 may be secured to the urine port 145 after the first conduit 117a is removed effective to prevent fluid communication with the interior region of the canister 114 through the urine port 144. In some embodiments, the first conduit 117a may be used to provide fluid communication between the cleaning solution container 260 and the dispenser port 143. In operation, when the lid 219 is secured to the canister 214, the additional conduit 217c is secured to the dispenser port 143 and the cleaning port 262, and the second conduit 117b is secured to the vacuum port 142, activation of the pump 216 creates the negative pressure in the interior region of the canister 214 effective to draw cleaning solution 155 in the cleaning solution container 260 through the additional conduit 217c, the dispenser port 143, and the one or more openings 154 of the dispenser 150 into the interior region of the canister 214.

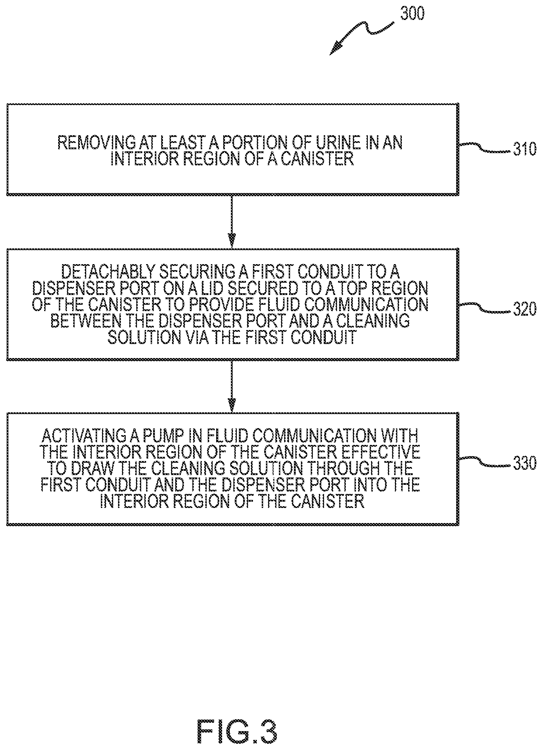

**FIG. 3** is a flow diagram of a method 300 of cleaning a canister of a urine collection system, such as the urine collection systems 100 or 200, according to an embodiment. The method may include an act 310 of removing at least a portion of urine in an interior region of a canister. The method also may include an act 320 of detachably securing a first conduit to a dispenser port on a lid secured to a top region of the canister to provide fluid communication between the dispenser port and a cleaning solution via the first conduit. The method also may include an act 330 of activating a pump in fluid communication with the interior region of the canister via a second conduit secured to a vacuum port on the lid, thereby creating a negative pressure in the interior region of the canister effective to draw the cleaning solution through the first conduit and the dispenser port into the interior region of the canister.

In some embodiments, the method 300 further includes an act of removing the first conduit or an additional conduit from a urine port on the lid, the first conduit or the additional conduit being in fluid communication with a urine collection device. The method 300 also may include an act of capping or closing the urine port on the lid after removing the first conduit or the additional conduit from the urine port. In some embodiments, the method 300 includes an act of, before removing the first conduit or the additional conduit from the urine port on the lid, drawing urine from the urine collection device through the first conduit or the additional conduit and into the interior region of the canister by capping dispenser port and activating the pump, thereby creating the negative pressure in the interior region of the canister effective to draw the urine from the urine collection device, through the additional conduit, and into the interior region of the canister. The method 300 also may include an act of, before activating the pump effective to draw the urine from the urine collection device, capping or closing the dispenser port.

In some embodiments, the act 330 of activating a pump thereby creating a negative pressure in the interior region of the canister effective to draw the cleaning solution through the first conduit and the dispenser port into the interior region of the canister may include activating the pump thereby creating the negative pressure in the interior region of the canister effective to draw the cleaning solution through the first conduit and the dispenser port and dispense the cleaning solution through one or more openings on a dispenser positioned in the interior region of the canister.

In some embodiments, the method 300 also may include acts of removing the second conduit from the vacuum port and releaseably securing the second conduit to a removal port on the lid. In these and other embodiments, the act 310 of removing at least a portion of urine in an interior region of a canister may include activating the pump effective to draw at least the portion of the urine in the interior region of the canister through an interior conduit positioned in the interior region, the removal port, and the second conduit. The method 300 may further include discharging at least the portion of the urine drawn from the interior region through a discharge port or opening on a base. In some embodiments, the method 300 includes removing a cap from the removal port and capping or closing the vacuum port after removing the second conduit form the vacuum port.

In some embodiments, the act 310 of removing at least a portion of urine in an interior region of a canister includes removing a cap from an opening on a bottom region of the canister to allow at least the portion of the urine in the interior region to empty from the canister.

The acts of the method 300 described above are for illustrative purposes. For example, the acts of the method 300 can be performed in different orders, split into multiple acts, modified, supplemented, or combined. In an embodiment, one or more of the act of the method 300 can be omitted from the method 300. Any of the acts of the method 300 can include using any of the urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments are disclosed within the scope of the appended set of claims.

## Claims

1. A portable urine collection system (100, 200), comprising:
a urine collection device (112) configured to be positioned at least proximate to a urethra of a user;
a canister (114, 214) having an interior region;
a base (115, 215) including a pump (116, 216);
a lid (119, 219) secured or securable to the canister (114, 214), the lid (119, 219) including a vacuum port (142), a urine port (144), and a dispenser port (143);
a cleaning solution container (160, 260) having a cleaning port (162, 262);
at least one first conduit (117a) having an outlet detachably secured or securable to the dispenser port (143) and/or the urine port (144), and an inlet detachably secured or securable to the urine collection device (112) and/or the cleaning port (162, 262);
a second conduit (117b) in fluid communication with the pump (116, 216) and having an inlet releasably securable to the vacuum port (142); and
a dispenser (150) in fluid communication with the dispenser port (143) and positioned or positionable in the interior region of the canister (114, 214), the dispenser (150) having one or more openings configured to mist or spray the cleaning solution into the interior region of the canister (114, 214);
wherein the urine collection system (100,200) is adjustable to adopt at least:
a urine collection configuration having the at least one first conduit (117a) secured to the urine collection device (112) and the urine port (144) and having the second conduit (117b) secured to the vacuum port (142) such that activation of the pump (116, 216) creates a negative pressure in the interior region of the canister (114, 214) effective to draw urine in the urine collection device (112) through the at least one first conduit (117a) into the interior region of the canister (114, 214); and
a cleaning configuration having the at least one first conduit (117a) secured to the dispenser port (143) and the cleaning port (162, 262), and having the second conduit (117b) secured to the vacuum port (142) such that activation of the pump (116, 216) creates the negative pressure in the interior region of the canister (114, 214) effective to draw cleaning solution in the cleaning solution container (160, 260) through the at least one first conduit (117a) and the dispenser port (143), and mist or spray the cleaning solution through the one or more openings of the dispenser (150) into the interior region of the canister (114, 214).

2. The portable urine collection system of claim 1, wherein the one or more openings configured to mist or spray the cleaning solution include multiple openings positioned to mist or spray the cleaning solution from multiple locations spaced from one another in the interior region of the canister (114, 214).

3. The portable urine collection system of claim 2, wherein, in the cleaning configuration activation of the pump (116, 216) creates the negative pressure in the interior region of the canister (114, 214) effective to draw cleaning solution in the cleaning solution container (160, 260) through the at least one first conduit (117a) and the one or more openings of the dispenser (150) into the interior region of the canister (114, 214).

4. The portable urine collection system of any of claims 2-3, wherein the multiple openings of the dispenser (150) include multiple spray nozzles spaced from one another in the interior region of the canister (114, 214).

5. The portable urine collection system of any of claims 1-4, wherein the cleaning solution container (160, 260) is secured or securable to at least one of the base (115, 215) or the canister (114, 214).

6. The portable urine collection system of any of claims 1-5, wherein the at least one first conduit (117a) includes one first conduit (117a) having the outlet selectively and detachably secured or securable to the dispenser port (143) and the urine port (144), and the inlet selectively and detachably secured or securable to the urine collection device (112) and the cleaning port (162, 262).

7. The portable urine collection system of any of claims 1-5, wherein the at least one first conduit (117a) includes:
a first conduit having an outlet detachably secured or securable to the urine port (144) and an inlet detachably secured or securable to the urine collection device (112); and
an additional first conduit having an outlet detachably secured or securable to the dispenser port (143) and an inlet secured or securable to the cleaning port (162, 262).

8. The portable urine collection system of any of claims 1-7, further comprising:
wherein the lid (119, 219) includes a removal port;
an interior conduit (152) secured or securable to the removal port to extend from the removal port at least partially into the interior region of the canister (114, 214);
a discharge port (118) or opening on the base (115, 215); and
wherein the urine collection system (100,200) is further adjustable to a urine removal configuration having the inlet of the second conduit (117b) selectively and detachably securable to the removal port such that activation of the pump (116, 216) draws fluid in the canister (114, 214) through the interior conduit (152) and the second conduit (117b), and discharges the fluid through the discharge port or opening on the base (115, 215).

9. The portable urine collection system of any of claims 1-7, wherein the canister (114, 214) includes a top region, a bottom region distal to the top region, an opening at the bottom region, and a cap detachably secured or securable to the opening.

10. The portable urine collection system of claim 9, wherein:
the base (115, 215) includes a recess (207) sized to receive at least a portion of the bottom region of the canister (114, 214) and a notch (217) positioned to provide access to the cap and/or the opening in the canister (114, 214) when the canister (114, 214) is positioned in the recess (207) of the base (115, 215); and
the urine collection system (100,200) is further adjustable to a urine removal configuration having the cap removed from the opening such that urine in the canisters (114, 214) empties from the canister (114, 214) through the opening.

11. A method of cleaning a canister (114, 214) of the portable urine collection system (100, 200) of any of claims 1-10, the method comprising:
removing at least a portion of urine in the interior region of the canister (114, 214);
detachably securing the first conduit (117a) to the dispenser port (143) on the lid (119, 219) secured to the top region of the canister (114, 214) to provide fluid communication between the dispenser port (143) and the cleaning solution via the first conduit (117a); and
activating the pump (116, 216) in fluid communication with the interior region of the canister (114, 214) via the second conduit (117b) secured to the vacuum port (142) on the lid (119, 219), thereby creating a negative pressure in the interior region of the canister (114, 214) effective to draw the cleaning solution through the first conduit (117a) and the dispenser port (143) and misting or spraying the cleaning solution into the interior region of the canister (114, 214)_through the one or more openings of the dispenser (150) positioned in the interior region of the canister (114, 214).

12. The method of claim 11, further comprising removing the additional conduit from the urine port (144) on the lid (119, 219), the additional conduit being in fluid communication with a urine collection device (112).

13. The method of claim 12, further comprising capping the urine port (144) on the lid (119, 219) after removing the additional conduit from the urine port (144).

14. The method of any of claims 12 or 13, further comprising, before removing the additional conduit from the urine port (144) on the lid (119, 219), drawing urine from the urine collection device (112) through the additional conduit and into the interior region of the canister (114, 214) by capping dispenser port (143) and activating the pump (116, 216), thereby creating the negative pressure in the interior region of the canister (114, 214) effective to draw the urine from the urine collection device (112), through the additional conduit, and into the interior region of the canister (114, 214).

15. The method of any of claims 11-14, wherein the cleaning solution is misted or sprayed through multiple openings on the dispenser at multiple locations spaced from one another in the interior region of the canister (114,214).

## Patentansprüche

1. Tragbares Urinsammelsystem (100, 200), umfassend:
eine Fluidsammelvorrichtung (112), die dazu konfiguriert ist, mindestens in der Nähe einer Harnröhre eines Benutzers positioniert zu werden;
einen Kanister (114, 214) mit einem Innenbereich; eine Basis (115, 215), die eine Pumpe (116, 216) beinhaltet;
einen Deckel (119, 219), der an dem Kanister (114, 214) befestigt oder befestigbar ist, wobei der Deckel (119, 219) einen Vakuumanschluss (142), einen Urinanschluss (144) und einen Spenderanschluss (143) beinhaltet;
einen Reinigungslösungsbehälter (160, 260) mit einem Reinigungsanschluss (162, 262);
mindestens eine erste Leitung (117a) mit einem Auslass, der abnehmbar an dem Spenderanschluss (143) und/oder dem Urinanschluss (144) befestigt oder befestigbar ist, und einem Einlass, der abnehmbar an der Urinsammelvorrichtung (112) und/oder dem Reinigungsanschluss (162, 262) befestigt oder befestigbar ist;
eine zweite Leitung (117b) in Fluidkommunikation mit der Pumpe (116, 216) und mit einem Einlass, der lösbar an dem Vakuumanschluss (142) befestigt werden kann; und
einen Spender (150) in Fluidkommunikation mit dem Spenderanschluss (143) und im Innenbereich des Kanisters (114, 214) positioniert oder positionierbar, wobei der Spender (150) eine oder mehrere Öffnungen aufweist, die konfiguriert sind, um die Reinigungslösung in den Innenbereich des Kanisters (114, 214) zu vernebeln oder zu sprühen;
wobei das Urinsammelsystem (100, 200) einstellbar ist, um mindestens einzunehmen:
eine Urinsammelkonfiguration mit der mindestens einen ersten Leitung (117a), die an der Urinsammelvorrichtung (112) und dem Urinanschluss (144) befestigt ist, und mit der zweiten Leitung (117b), die an dem Vakuumanschluss (142) befestigt ist, so dass eine Aktivierung der Pumpe (116, 216) einen Unterdruck in dem Innenbereich des Kanisters (114, 214) erzeugt, der wirksam ist, um Urin in der Urinsammelvorrichtung (112) durch die mindestens eine erste Leitung (117a) in den Innenbereich des Kanisters (114, 214) anzusaugen; und
eine Reinigungskonfiguration mit der mindestens einen ersten Leitung (117a), die an dem Spenderanschluss (143) und dem Reinigungsanschluss (162, 262) befestigt ist, und mit der zweiten Leitung (117b), die an dem Vakuumanschluss (142) befestigt ist, so dass eine Aktivierung der Pumpe (116, 216) den Unterdruck in dem Innenbereich des Kanisters (114, 214) erzeugt, der wirksam ist, um Reinigungslösung in dem Reinigungslösungsbehälter (160, 260) durch die mindestens eine erste Leitung (117a) und den Spenderanschluss (143) anzusaugen und die Reinigungslösung durch die eine oder mehreren Öffnungen des Spenders (150) in den Innenbereich des Kanisters (114, 214) zu vernebeln oder zu sprühen.

2. Tragbares Urinsammelsystem nach Anspruch 1, wobei die eine oder mehreren Öffnungen, die konfiguriert sind, um die Reinigungslösung zu vernebeln oder zu sprühen, mehrere Öffnungen beinhalten, die positioniert sind, um die Reinigungslösung von mehreren Stellen, die voneinander beabstandet sind, in den Innenbereich des Kanisters (114, 214) zu vernebeln oder zu sprühen.

3. Tragbares Urinsammelsystem nach Anspruch 2, wobei in der Reinigungskonfiguration die Aktivierung der Pumpe (116, 216) den Unterdruck in dem Innenbereich des Kanisters (114, 214) erzeugt, der wirksam ist, um Reinigungslösung in dem Reinigungslösungsbehälter (160, 260) durch die mindestens eine erste Leitung (117a) und die eine oder mehreren Öffnungen des Spenders (150) in den Innenbereich des Kanisters (114, 214) anzusaugen.

4. Tragbares Urinsammelsystem nach einem der Ansprüche 2-3, wobei die mehreren Öffnungen des Spenders (150) mehrere Sprühdüsen beinhalten, die im Innenbereich des Kanisters (114, 214) voneinander beabstandet sind.

5. Tragbares Urinsammelsystem nach einem der Ansprüche 1-4, wobei der Reinigungslösungsbehälter (160, 260) an mindestens der Basis (115, 215) und/oder dem Kanister (114, 214) befestigt oder befestigbar ist.

6. Tragbares Urinsammelsystem nach einem der Ansprüche 1-5, wobei die mindestens eine erste Leitung (117a) eine erste Leitung (117a) beinhaltet, bei der der Auslass selektiv und abnehmbar an dem Spenderanschluss (143) und dem Urinanschluss (144) befestigt oder befestigbar ist und der Einlass selektiv und abnehmbar an der Urinsammelvorrichtung (112) und dem Reinigungsanschluss (162, 262) befestigt oder befestigbar ist.

7. Tragbares Urinsammelsystem nach einem der Ansprüche 1-5, wobei die mindestens eine erste Leitung (117a) Folgendes beinhaltet:
eine erste Leitung mit einem Auslass, der abnehmbar an dem Urinanschluss (144) befestigt oder befestigbar ist, und einem Einlass, der abnehmbar an der Urinsammelvorrichtung (112) befestigt oder befestigbar ist; und
eine zusätzliche erste Leitung mit einem Auslass, der abnehmbar an dem Spenderanschluss (143) befestigt oder befestigbar ist, und einem Einlass, der an dem Reinigungsanschluss (162, 262) befestigt oder befestigbar ist.

8. Tragbares Urinsammelsystem nach einem der Ansprüche 1-7, ferner umfassend:
wobei der Deckel (119, 219) einen Entnahmeanschluss beinhaltet;
eine Innenleitung (152), die an dem Entnahmeanschluss befestigt oder befestigbar ist, um sich von dem Entnahmeanschluss zumindest teilweise in den Innenbereich des Kanisters (114, 214) zu erstrecken;
einen Auslassanschluss (118) oder eine Öffnung an der Basis (115, 215); und
wobei das Urinsammelsystem (100, 200) ferner auf eine Urinentnahmekonfiguration einstellbar ist, bei der der Einlass der zweiten Leitung (117b) selektiv und abnehmbar an dem Entnahmeanschluss befestigbar ist, sodass eine Aktivierung der Pumpe (116, 216) Fluid in dem Kanister (114, 214) durch die Innenleitung (152) und die zweite Leitung (117b) ansaugt und das Fluid durch den/die Auslassanschluss oder -öffnung an der Basis (115, 215) abgibt.

9. Tragbares Urinsammelsystem nach einem der Ansprüche 1-7, wobei der Kanister (114, 214) einen oberen Bereich, einen unteren Bereich distal zu dem oberen Bereich, eine Öffnung an dem unteren Bereich und eine Kappe, die abnehmbar an der Öffnung befestigt oder befestigbar ist, beinhaltet.

10. Tragbares Urinsammelsystem nach Anspruch 9, wobei:
die Basis (115, 215) eine Aussparung (207), die bemessen ist, um mindestens einen Abschnitt des unteren Bereichs des Kanisters (114, 214) aufzunehmen, und eine Kerbe (217) beinhaltet, die positioniert ist, um Zugang zu der Kappe und/oder der Öffnung in dem Kanister (114, 214) bereitzustellen, wenn der Kanister (114, 214) in der Aussparung (207) der Basis (115, 215) positioniert ist; und
das Urinsammelsystem (100, 200) ferner auf eine Urinentnahmekonfiguration einstellbar ist, bei der die Kappe von der Öffnung entfernt ist, sodass Urin in den Kanistern (114, 214) durch die Öffnung aus dem Kanister (114, 214) entleert wird.

11. Verfahren zum Reinigen eines Kanisters (114, 214) des tragbaren Urinsammelsystems (100, 200) nach einem der Ansprüche 1-10, wobei das Verfahren Folgendes umfasst:
Entnehmen mindestens eines Teils des Urins im Innenbereich des Kanisters (114, 214);
abnehmbares Befestigen der ersten Leitung (117a) an dem Spenderanschluss (143) auf dem Deckel (119, 219), der an dem oberen Bereich des Kanisters (114, 214) befestigt ist, um eine Fluidkommunikation zwischen dem Spenderanschluss (143) und der Reinigungslösung über die erste Leitung (117a) bereitzustellen; und
Aktivieren der Pumpe (116, 216) in Fluidkommunikation mit dem Innenbereich des Kanisters (114, 214) über die zweite Leitung (117b), die an dem Vakuumanschluss (142) auf dem Deckel (119, 219) befestigt ist, wodurch ein Unterdruck in dem Innenbereich des Kanisters (114, 214) erzeugt wird, der wirksam ist, um die Reinigungslösung durch die erste Leitung (117a) und den Spenderanschluss (143) anzusaugen, und Vernebeln oder Sprühen der Reinigungslösung in den Innenbereich des Kanisters (114, 214) durch die eine oder mehreren Öffnungen des Spenders (150), die in dem Innenbereich des Kanisters (114, 214) positioniert sind.

12. Verfahren nach Anspruch 11, ferner umfassend das Entfernen der zusätzlichen Leitung von dem Urinanschluss (144) auf dem Deckel (119, 219), wobei die zusätzliche Leitung in Fluidkommunikation mit einer Urinsammelvorrichtung (112) steht.

13. Verfahren nach Anspruch 12, ferner umfassend das Verschließen des Urinanschlusses (144) auf dem Deckel (119, 219) nach dem Entfernen der zusätzlichen Leitung von dem Urinanschluss (144).

14. Verfahren nach einem der Ansprüche 12 oder 13, ferner umfassend, vor dem Entfernen der zusätzlichen Leitung von dem Urinanschluss (144) auf dem Deckel (119, 219), Ansaugen von Urin von der Urinsammelvorrichtung (112) durch die zusätzliche Leitung und in den Innenbereich des Kanisters (114, 214) durch Verschließen des Spenderanschlusses (143) und Aktivieren der Pumpe (116, 216), wodurch der Unterdruck in dem Innenbereich des Kanisters (114, 214) erzeugt wird, der wirksam ist, um den Urin von der Urinsammelvorrichtung (112) durch die zusätzliche Leitung und in den Innenbereich des Kanisters (114, 214) anzusaugen.

15. Verfahren nach einem der Ansprüche 11-14, wobei die Reinigungslösung durch mehrere Öffnungen an dem Spender an mehreren voneinander beabstandeten Stellen in dem Innenbereich des Kanisters (114, 214) vernebelt oder versprüht wird.

## Revendications

1. Système portable de collecte d'urine (100, 200), comprenant :
un dispositif de collecte d'urine (112) configuré pour être positionné au moins à proximité de l'urètre d'un utilisateur ;
un récipient (114, 214) ayant une zone intérieure ; une base (115, 215) comprenant une pompe (116, 216) ;
un couvercle (119, 219) fixé ou pouvant être fixé au récipient (114, 214), le couvercle (119, 219) comprenant un orifice d'aspiration (142), un orifice d'urine (144) et un orifice de distribution (143) ;
un récipient de solution de nettoyage (160, 260) ayant un orifice de nettoyage (162, 262) ;
au moins un premier conduit (117a) ayant une sortie fixée ou pouvant être fixée de manière amovible à l'orifice de distribution (143) et/ou à l'orifice d'urine (144), et une entrée fixée ou pouvant être fixée de manière amovible au dispositif de collecte d'urine (112) et/ou à l'orifice de nettoyage (162, 262) ;
un second conduit (117b) en communication fluidique avec la pompe (116, 216) et dont l'entrée peut être fixée de manière amovible à l'orifice d'aspiration (142) ; et
un distributeur (150) en communication fluidique avec l'orifice de distribution (143) et positionné ou pouvant être positionné dans la zone intérieure du récipient (114, 214), le distributeur (150) ayant une ou plusieurs ouvertures configurées pour vaporiser ou pulvériser la solution de nettoyage dans la zone intérieure du récipient (114, 214) ;
dans lequel le système de collecte d'urine (100, 200) est réglable pour adopter au moins :
une configuration de collecte d'urine ayant l'au moins un premier conduit (117a) fixé au dispositif de collecte d'urine (112) et à l'orifice d'urine (144) et ayant le second conduit (117b) fixé à l'orifice d'aspiration (142) de sorte que l'activation de la pompe (116, 216) crée une pression négative dans la zone intérieure du récipient (114, 214) efficace pour aspirer l'urine dans le dispositif de collecte d'urine (112) à travers l'au moins un premier conduit (117a) dans la zone intérieure du récipient (114, 214) ; et
une configuration de nettoyage dans laquelle l'au moins un premier conduit (117a) est fixé à l'orifice de distribution (143) et à l'orifice de nettoyage (162, 262), et le second conduit (117b) est fixé à l'orifice d'aspiration (142) de sorte que l'activation de la pompe (116, 216) crée une pression négative dans la zone intérieure du récipient (114, 214) efficace pour aspirer la solution de nettoyage dans le conteneur de solution de nettoyage (160, 260) à travers l'au moins un premier conduit (117a) et l'orifice de distributeur (143), et vaporiser ou pulvériser la solution de nettoyage à travers une ou plusieurs ouvertures du distributeur (150) dans la zone intérieure du récipient (114, 214).

2. Système portable de collecte d'urine selon la revendication 1, dans lequel l'une ou plusieurs ouvertures configurées pour vaporiser ou pulvériser la solution de nettoyage comprennent plusieurs ouvertures positionnées pour vaporiser ou pulvériser la solution de nettoyage à partir de plusieurs endroits espacés les uns des autres dans la zone intérieure du récipient (114, 214).

3. Système portable de collecte d'urine selon la revendication 2, dans lequel, dans la configuration de nettoyage, l'activation de la pompe (116, 216) crée une pression négative dans la zone intérieure du récipient (114, 214) efficace pour aspirer la solution de nettoyage dans le récipient de solution de nettoyage (160, 260) à travers l'au moins un premier conduit (117a) et l'une ou plusieurs ouvertures du distributeur (150) dans la zone intérieure du récipient (114, 214).

4. Système portable de collecte d'urine selon l'une quelconque des revendications 2 à 3, dans lequel les multiples ouvertures du distributeur (150) comprennent plusieurs buses de pulvérisation espacées les unes des autres dans la zone intérieure du récipient (114, 214).

5. Système portable de collecte d'urine selon l'une quelconque des revendications 1 à 4, dans lequel le récipient de solution de nettoyage (160, 260) est fixé ou pouvant être fixé à au moins l'une des deux bases (115, 215) ou au récipient (114, 214).

6. Système portable de collecte d'urine selon l'une quelconque des revendications 1 à 5, dans lequel l'au moins un premier conduit (117a) comprend un premier conduit (117a) dont la sortie est sélectivement fixée ou pouvant être fixée de manière amovible à l'orifice de distribution (143) et à l'orifice d'urine (144), et dont l'entrée est sélectivement fixée ou pouvant être fixée de manière amovible au dispositif de collecte d'urine (112) et à l'orifice de nettoyage (162, 262).

7. Système portable de collecte d'urine selon l'une quelconque des revendications 1 à 5, dans lequel au moins un premier conduit (117a) comprend :
un premier conduit ayant une sortie fixée ou pouvant être fixée de manière amovible à l'orifice d'urine (144) et une entrée fixée ou pouvant être fixée de manière amovible au dispositif de collecte d'urine (112) ; et
un premier conduit supplémentaire ayant une sortie fixée ou pouvant être fixée de manière amovible à l'orifice de distribution (143) et une entrée fixée ou pouvant être fixée à l'orifice de nettoyage (162, 262).

8. Système portable de collecte d'urine selon l'une quelconque des revendications 1 à 7, comprenant en outre :
dans lequel le couvercle (119, 219) comprend un orifice de retrait ;
un conduit intérieur (152) fixé ou pouvant être fixé à l'orifice de retrait pour s'étendre de l'orifice de retrait au moins partiellement dans la zone intérieure du récipient (114, 214) ;
un orifice de décharge (118) ou une ouverture sur la base (115, 215) ; et
dans lequel le système de collecte d'urine (100, 200) est en outre réglable à une configuration de retrait d'urine ayant l'entrée du second conduit (117b) sélectivement fixée de manière amovible de l'orifice de retrait de sorte que l'activation de la pompe (116, 216) aspire le fluide dans le récipient (114, 214) à travers le conduit intérieur (152) et le second conduit (117b), et décharge le fluide à travers l'orifice de décharge ou l'ouverture sur la base (115, 215).

9. Système portable de collecte d'urine selon l'une quelconque des revendications 1 à 7, dans lequel le récipient (114, 214) comprend une zone supérieure, une zone inférieure distale de la zone supérieure, une ouverture dans la zone inférieure et un capuchon fixé ou pouvant être fixé de manière amovible à l'ouverture.

10. Système portable de collecte d'urine selon la revendication 9, dans lequel :
la base (115, 215) comprend un renfoncement (207) dimensionné pour recevoir au moins une partie de la zone inférieure du récipient (114, 214) et une encoche (217) positionnée pour permettre l'accès au capuchon et/ou à l'ouverture du récipient (114, 214) lorsque le récipient (114, 214) est positionné dans le renfoncement (207) de la base (115, 215) ; et
le système de collecte d'urine (100, 200) est en outre réglable dans une configuration de retrait de l'urine dans laquelle le capuchon est retiré de l'ouverture, de sorte que l'urine contenue dans les récipients (114, 214) se vide du récipient (114, 214) par l'ouverture.

11. Procédé de nettoyage d'un récipient (114, 214) du système portable de collecte d'urine (100, 200) selon l'une quelconque des revendications 1 à 10, le procédé comprenant :
le retrait d'au moins une partie de l'urine dans la zone intérieure du récipient (114, 214) ;
la fixation de manière amovible du premier conduit (117a) à l'orifice de distribution (143) sur le couvercle (119, 219) fixé à la zone supérieure du récipient (114, 214) pour assurer la communication fluidique entre l'orifice de distribution (143) et la solution de nettoyage par l'intermédiaire du premier conduit (117a) ; et
l'activation de la pompe (116, 216) en communication fluidique avec la zone intérieure du récipient (114, 214) par l'intermédiaire du second conduit (117b) fixé à l'orifice d'aspiration (142) sur le couvercle (119, 219), créant ainsi une pression négative dans la zone intérieure du récipient (114, 214) efficace pour aspirer la solution de nettoyage à travers le premier conduit (117a) et l'orifice de distributeur (143) et pour vaporiser ou pulvériser la solution de nettoyage dans la zone intérieure du récipient (114, 214) _à travers une ou plusieurs ouvertures du distributeur (150) positionné dans la zone intérieure du récipient (114, 214).

12. Procédé selon la revendication 11, comprenant en outre le retrait du conduit supplémentaire de l'orifice d'urine (144) sur le couvercle (119, 219), le conduit supplémentaire étant en communication fluidique avec un dispositif de collecte d'urine (112).

13. Procédé selon la revendication 12, comprenant en outre le bouchage de l'orifice d'urine (144) sur le couvercle (119, 219) après avoir retiré le conduit supplémentaire de l'orifice d'urine (144).

14. Procédé selon l'une quelconque des revendications 12 ou 13, comprenant en outre, avant de retirer le conduit supplémentaire de l'orifice d'urine (144) sur le couvercle (119, 219), l'aspiration de l'urine du dispositif de collecte d'urine (112) à travers le conduit supplémentaire et dans la zone intérieure du récipient (114, 214) en bouchant l'orifice de distributeur (143) et en activant la pompe (116, 216), créant ainsi une pression négative dans la zone intérieure du récipient (114, 214) efficace pour aspirer l'urine du dispositif de collecte d'urine (112), à travers le conduit supplémentaire, et dans la zone intérieure du récipient (114, 214).

15. Procédé selon l'une quelconque des revendications 11 à 14, dans lequel la solution de nettoyage est vaporisée ou pulvérisée à travers plusieurs ouvertures sur le distributeur à plusieurs endroits espacés les uns des autres dans la zone intérieure du récipient (114, 214).
